# EUROPEAN PATENT SPECIFICATION

(11) **EP 2 946 771 B1**
(45) Date of publication and mention of the grant of the patent: **20.03.2019**
(21) Application number: 15167950.3
(22) Date of filing: 18.05.2015
(51) Int. Cl.: A61K 9/20, A61K 31/41

(54) **WATER-DISPERSIBLE TABLET FORMULATION COMPRISING DEFERASIROX**
WASSERDISPERGIERBARE TABLETTENFORMULIERUNG MIT DEFERASIROX
FORMULATION DE COMPRIMÉ DISPERSIBLE DANS L'EAU COMPRENANT DU DÉFÉRASIROX

(30) Priority: 20.05.2014 TR 201405598
(43) Date of publication of application: 25.11.2015
(73) Proprietor: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Yildirim, Ediz, 34460 Istanbul (TR); Karabulut, Tutku Ceren, 34460 Istanbul (TR)
(74) Representative: Sevinç, Erkan

(56) References cited:
- EP-A1- 2 062 572
- WO-A1-2010/035282

## Description

### Field of Invention

The present invention relates to a formulation comprising deferasirox or a pharmaceutically acceptable salt thereof. More particularly, the present invention relates to a water-dispersible tablet formulation of deferasirox.

### Background of Invention

Deferasirox is an iron-binding agent used for the treatment of iron overload. Its chemical name is 4-[3,5-bis(2-hydroxyphenyl)[1,2,4]triazole-1-yl]benzoic acid and has the structure shown below in formula I.

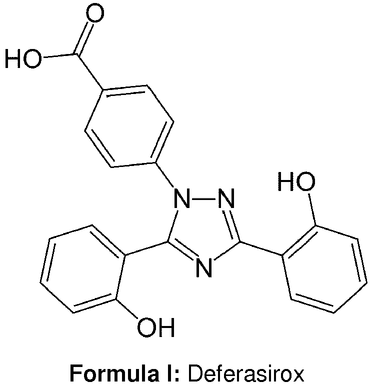

Deferasirox is indicated in the treatment of iron overload for reducing iron-related morbidity and mortality in transfusion-dependent anemia, particularly in mild and severe thalassemia, and in sickle cell anemia. At the same time, deferasirox can be used in the treatment of hemochromatosis. The red blood cells in the body of patients afflicted with mild and severe thalassemia, sickle-cell anemia, and hemochromatosis are exposed to a very rapid destruction and a significant amount of blood transfusion is given to the patients in the need thereof. If the excessive amount of iron following a blood transfusion is not eliminated from the body, it may cause organ failures. Deferasirox is an effective active agent in eliminating excessive iron from the body.

Deferasirox is the first oral drug approved for the treatment of iron overload in the United States. It was approved by FDA (Food and Drug Administration) in November 2005.

Deferasirox, which is commercially-available in water-dispersible forms (EXJADE®) was first disclosed in the patent WO97/49395, wherein it was indicated for the treatment of diseases caused by excessive iron in the body.

Various water-dispersible tablet formulations comprising deferasirox can be found in the prior art.

The patent application EP1734924A1 of Novartis, for instance, discloses a dispersible tablet comprising deferasirox in an amount of 42% to 65% by weight of the total tablet.

The patent application EP1940360 of Novartis, in turn, discloses a dispersible tablet formulation comprising deferasirox in an amount of 42% to 65% by weight based on the total weight of the tablet, and at least one excipient.

The patent TR2011/03691 of the firm Ali Raif discloses a water-dispersible tablet formulation comprising deferasirox in an amount above 40,5% and below 41,55% of the total weight of the tablet.

The patent TR2009/05371 of Novartis discloses a dispersible tablet formulation comprising deferasirox in an amount of 5% to 40% by weight based on the total weight of the tablet and a disintegrant in an amount of 10% to 35% by weight based on the total weight of the tablet. That document further discloses a complex production method consisting of 4 steps. The patents and patent applications according to the prior art referred to above disclose similar production methods. Therefore, a simple, time-saving and cost-efficient production method would be desirable.

125 mg, 250 mg and 500 mg tablet formulations of deferasirox are available, but particularly since the total tablet weight of the 500 mg dose is too high, it is not suitable for use as oral tablets. It is known that administering oral tablets with a large tablet size is problematic in patients with difficulty swallowing. The water-dispersible tablet formulations comprising deferasirox have been developed for this reason.

The term "water-dispersible tablet" refers to a tablet, which is administered after it is dissolved in water.

WO 2010/035282 A1 relates to oral pharmaceutical compositions composition comprising deferasirox or pharmaceutically acceptable salts, polymorphic forms and mixtures thereof, wherein the active ingredient has a mean particle size less than about 100µm and is present in an amount greater than 66% by weight based on total weight of the tablet.

It is known that deferasirox is poorly soluble in water. For this reason, the most-frequently encountered difficulty in producing water-dispersible tablets comprising deferasirox is the solubility thereof.

Additionally, the particles obtained in the production of dispersible tablets of deferasirox have low flow properties and are prone to adhere to each other because of the low density and the electrostatic characteristics of deferasirox.

In order to fulfill all the requirements described above, a special drug formulation should be adapted particularly by a careful selection of the excipients to be used. Therefore, water-dispersible tablets comprising deferasirox are desired, which would not lead to losses in the active agents or excipients during production, i.e. would have a high production yield, would rapidly dissolve in water, have good flow properties, and would be simply compressed into tablets. Other advantages and embodiments of the present invention will be clarified in the following description.

### Detailed Description of Invention

The main object of the present invention is to provide a water-dispersible tablet formulation comprising deferasirox or a pharmaceutically acceptable salt thereof according to claim 1 which overcomes the problems referred to above using suitable excipients.

Another object of the present invention is to provide a water-dispersible tablet formulation which does not lead to losses in the active agents or excipients during production, i.e. has a high production yield.

A further object of the present invention is to provide a water-dispersible tablet formulation comprising a pharmaceutically effective amount of deferasirox and having a desired level of solubility and dissolution rate.

A further object of the present invention is to provide a water-dispersible tablet formulation of deferasirox or a pharmaceutically acceptable salt thereof, which has suitable mechanical strength (e.g. adequate hardness and low erosion) to be effectively processed in high-speed tablet presses and transported in low-cost packages.

In terms of the prior art, the most-frequently used glidant in water-soluble tablet formulations is colloidal silicon dioxide. It optimizes the flow properties of powders used in such formulations and simplifies their direct compression. Colloidal silicon dioxide has small particles such that these small particles surround the larger particles and reduce the Van der Waals forces in between them. It also absorbs the moisture on the surface of hygroscopic powders and thus reduces the agglomeration and aggregation of the powders. Colloidal silicon dioxide enhances the flow properties and provides dose uniformity of the tablet, prevents the agglomeration which is highly probable to take place during the filling process and thus improves some properties of the tablet such as erosion and hardness.

It is further known that all colloidal silicon dioxide is provided in the powder phase of the tablets comprising powder and granule phases in the formulations according to the prior art. The mixture consisting of deferasirox, lactose, crospovidone prepared for the granulation step, the first step of the production method, is free of colloidal silicon dioxide. No sieving is carried out here, since this phase which is free of colloidal silicon dioxide adheres to the sieve and occludes the pores thereof. On the other hand, in the present invention, 50% by weight of colloidal silicon dioxide is provided in the granular phase of the tablet, whereas 50% thereof is provided in the powder phase thereof. The problem referred to above was surprisingly overcome by adding half of the total weight of colloidal silicon dioxide to the deferasirox-comprising powder phase prepared for the granulation step and it was possible to carry out sieving. Thus, a process was achieved which has high production yield, is simple, time-saving and cost-efficient.

The term "granular phase of the tablet" refers to the part of the tablet in which the active agents or excipients used in tablet production are provided in a granulated form, i.e. after they are subjected to granulation.

The term "powder phase of the tablet" refers to the part of the tablet in which the active agents or excipients used in tablet production are provided in powder form.

No binder is used in the granulation solvent according to the present invention and the granulation step is carried out using only water. According to the prior art, however, polyvinylpyrrolidone and water are used as the granulation solvent. The greatest disadvantage of carrying out the granulation step using a binder-water mixture is that some part of the solution sticks to the wall of the container in which granulation is performed, leading to a loss in the amount of the binder used. It was found that this problem is avoided when granulation is carried out using water only and that the granulation step can be performed via a simpler process. Thus, a process was achieved which has a high production yield. Additionally, in case an early granule formation takes place, the granulation solvent cannot be used in whole, some part of the binder remains unused and therefore the amount of the binder used in the formulation can vary. In the present invention, in turn, the granulation step was carried using only water so that a granulation solvent was obtained which does not depend on the amount of binder.

A further object of the present invention is to provide highly-soluble water-dispersible tablets comprising deferasirox or a pharmaceutically acceptable salt thereof. Accordingly, it was surprisingly found that using sodium lauryl sulfate in the powder phase of the tablet in an amount of 3.00% to 5.00% of the total weight of the tablet increases the solubility of the formulation. Since the percentage amount of deferasirox is high according to the present invention, the tablet cannot be dispersed in water completely when sodium lauryl sulfate is used below this limit, and when it is used above this limit, it causes the formulation to foam. Sodium lauryl sulfate is an anionic surfactant having a hydrophobic and a hydrophilic group. Based on this structural property, it is a surfactant which is very effective in increasing the solubility of active agents such as deferasirox which do not dissolve in water. The hydrophobic groups of sodium lauryl sulfate surround the deferasirox molecule and prevent the contact of deferasirox molecules with water. Only the hydrophilic groups of sodium lauryl sulfate contact with water and makes deferasirox dissolve in water.

Another object of the present invention is to provide tablets with suitable hardness and low friability, as well as a simple and time-saving process for this purpose. The binder used in the present invention was polyvinylpyrrolidone-vinyl acetate copolymer in the powder phase of the tablet. Polyvinylpyrrolidone-vinyl acetate copolymer has a structure which is similar to sodium lauryl sulfate. The polyvinylpyrrolidone part of the copolymer is hydrophilic, whereas the vinyl acetate part thereof is hydrophobic. By virtue of this structure, the polyvinylpyrrolidone-vinyl acetate copolymer provided a synergistic effect together with sodium lauryl sulfate in the water-solubility of the tablet. By virtue of the hydrophobic and hydrophilic groups, it is a more suitable binder than others for the water-dispersible tablet formulations of deferasirox. Additionally, the most important feature of the polyvinylpyrrolidone-vinyl acetate copolymer that distinguishes it from polyvinylpyrrolidone is its plasticity. By virtue of the polyvinylpyrrolidone-vinyl acetate copolymer, the products obtained during tabletting are less prone to capping and the tablets are less friable. For these reasons, the polyvinylpyrrolidone-vinyl acetate copolymer is such a binder that is more suitable for the direct compression method over the binders such as polyvinylpyrrolidone and cellulose derivatives, and whilst it provides the required hardness for direct compression, it reduces the friability of the tablets. It was possible to press the tablets at 60 - 140 N by using the polyvinylpyrrolidone-vinyl acetate copolymer in the powder phase of the tablet in an amount of 1.00% to 3.00% of the total weight of the tablet.

Furthermore, crospovidone was used in the present invention as a disintegrant both in the powder phase and in the granular phase of the tablet to increase the solubility of the water-dispersible tablet. The proportion of crospovidone in the powder phase to sodium lauryl sulfate in the powder phase was held between 1:1 and 10:1, preferably between 3:1 and 7:1. By setting the proportion of crospovidone to sodium lauryl sulfate between 1:1 and 10:1, preferably between 3:1 and 7:1, a better dissolution profile was achieved. Additionally, since more homogenous solutions are obtained using tablets which disintegrate well in water, the patient compliance to the treatment was also improved.

Fillers which may be used in the water-dispersible tablet according to the present invention may be sorbitol, sucrose, inorganic salts, calcium salts, microcrystalline cellulose, dextrose, dicalcium phosphate, sodium chloride, dextrates, lactitol, maltodextrin, sucrose-maltodextrin mixtures, xylitol, trehalose, lactose, lactose monohydrate, spray-dried lactose monohydrate, or a mixture thereof. The water-dispersible tablet formulation according to the present invention comprises a filler or fillers in an amount of 25.00% to 31.00% of the total weight of the tablet. Microcrystalline cellulose and spray-dried lactose monohydrate are used as fillers.

Lubricants which may be used in the water-dispersible tablet according to the present invention may be sodium stearyl fumarate, sodium lauril sulfate, magnesium lauryl sulfate, magnesium stearate, fumaric acid, sodium chloride benzoate, sodium chloride acetate, glyceryl palmitostearate, hydrogenated natural oils, zinc stearate, calcium stearate, silica, talk, stearic acid, polyethylene glycol, or paraffin. The lubricant used is preferably magnesium stearate. The water-dispersible tablet formulation according to the present invention comprises magnesium stearate in an amount of 0.10% to 1.00% of the total weight of the tablet.

The granular phase of the tablet comprises deferasirox, 50% by weight of the total amount of colloidal silicon dioxide, and 55% by weight of the total amount of crospovidone.

The powder phase of the tablet comprises 50% by weight of the total amount of colloidal silicon dioxide, sodium lauryl sulfate, polyvinylpyrrolidone-vinyl acetate copolymer, 45% by weight of the total amount of crospovidone, spray-dried lactose monohydrate, microcrystalline cellulose and magnesium stearate.

The water-dispersible tablet according to the present invention was obtained by a formulation according to claim 9 and comprising the following ingredients:
a. granular phase:
   i. deferasirox in an amount of 26.00% to 29.00% of the total weight of the tablet,
   ii. crospovidone in an amount of 18.00% to 21.00% of the total weight of the tablet,
   iii. colloidal silicon dioxide in an amount of 0.01 to 0.50% of the total weight of the tablet,
b. powder phase:
   i. crospovidone in an amount of 17.00% to 20.00% of the total weight of the tablet,
   ii. lactose monohydrate (spray-dried) in an amount of 8.00% to 11.00% of the total weight of the tablet,
   iii. microcrystalline cellulose in an amount of 17.00% to 20.00% of the total weight of the tablet,
   iv. polyvinylpyrrolidone-vinyl acetate copolymer in an amount of 1.00% to 3.00% of the total weight of the tablet,
   v. sodium lauryl sulfate in an amount of 3.00% to 5.00% of the total weight of the tablet,
   vi. colloidal silicon dioxide in an amount of 0.01 to 0.50% of the total weight of the tablet,
   vii. magnesium stearate in an amount of 0.10% to 1.00% of the total weight of the tablet, where the granular phase of the tablet does not include a binder.

When the present invention was practiced via a production method according to claim 10 comprising the steps of
i. granulating deferasirox, 50.0% by weight of the total amount of colloidal silicon dioxide, and at least one excipient using water, where said excipient is not a binder,
ii. drying the wet granules obtained according to step (i) in a drying oven to give the granular phase of the tablet,
iii. mixing 50.0% by weight of the total amount of colloidal silicon dioxide and at least one excipient together to give the powder phase of the tablet, and
iv. adding the powder phase of step (iii) to the granular phase of step (ii), it was observed that the powders making up the granular phase did not occlude the pores of the sieve during sieving.

When the present invention was practiced via a production method according to claim 11 comprising the steps of
i. granulating deferasirox, 50.0% by weight of the total amount of colloidal silicon dioxide, and at least one excipient using water,
ii. drying the wet granules obtained according to step (i) in a drying oven to give the granular phase of the tablet,
iii. mixing 50.0% by weight of the total amount of colloidal silicon dioxide, sodium lauryl sulfate, polyvinylpyrrolidone-vinyl acetate copolymer and at least one excipient together to give the powder phase,
iv. adding the powder phase of step (iii) to the granular phase of step (ii),
it was observed that the powders making up the granular phase did not occlude the pores of the sieve during sieving. The tablets obtained further had high solubility, suitable hardness and low friability.

A preferred method according to the present invention for preparing a water-dispersible tablet formulation comprises the following steps, wherein
a) weighed amounts of deferasirox, 50% by weight of the total amount of colloidal silicon dioxide and 55% by weight of the total amount of crospovidone are taken into a granulator and stirred at a low speed for 10 minutes,
b) this mixture is sieved through a 630 µm sieve and taken into a mixer, then stirred for 5 minutes,
c) water prepared as the granulation solvent is added to the powder mixture in the granulator to complete the granulation process,
d) wet granules are sieved through a sieve and spread over a tray, then dried in a drying oven,
e) dried granules are sieved to give the granular phase,
f) 45% by weight of the total amount of crospovidone, spray-dried lactose monohydrate, microcrystalline cellulose, polyvinylpyrrolidone-vinyl acetate copolymer, sodium lauryl sulfate and 50% by weight of the total amount of colloidal silicon dioxide are sieved and stirred to give the powder phase,
g) the granular phase and powder phase of the tablet are mixed, then magnesium stearate is sieved and added into the mixture and stirred for 2 more minutes,
h) using the homogeneous mixture obtained above, tablets are compressed in a hardness range of 60 - 140 N.

### Example 1: Water-dispersible tablet comprising deferasirox

| **Ingredients** | **Amount (%)** |
|---|---|
| ***Granular phase*** | |
| Deferasirox | 26.00 - 29.00 |
| Crospovidone | 18.00 - 21.00 |
| Colloidal silicon dioxide | 0.01 - 0.50 |

| ***Powder phase*** | |
|---|---|
| Crospovidone | 17.00 - 20.00 |
| Spray-dried lactose monohydrate | 8.00 - 11.00 |
| Microcrystalline cellulose | 17.00 - 20.00 |
| Polyvinylpyrrolidone-vinyl acetate copolymer | 1.00 - 3.00 |
| Sodium lauryl sulfate | 3.00 - 5.00 |
| Colloidal silicon dioxide | 0.01 - 0.50 |
| Magnesium stearate | 0.1 - 1.00 |

### Production method:

→ Weighed amounts of deferasirox, 50% by weight of the total amount of colloidal silicon dioxide and 55% by weight of the total amount of crospovidone are taken into a granulator and stirred at a low speed for 10 minutes.
→ This mixture is sieved through a 630 µm sieve and taken into a mixer, stirred for 5 minutes.
→ Water which is prepared as the granulation solvent is added to the powder mixture in the granulator to complete the granulation process.
→ Wet granules are sieved through a sieve, spread over trays, and dried in a drying oven to give the granular phase of the tablet.
→ 45% by weight of the total amount of crospovidone, spray-dried lactose monohydrate, microcrystalline cellulose, polyvinylpyrrolidone-vinyl acetate copolymer, sodium lauryl sulfate and 50% by weight of the total amount of colloidal silicon dioxide are sieved and mixed to give the powder phase of the tablet.
→ The granular and powder phases of the tablet are stirred, then magnesium stearat is sieved and added to the mixture and stirred for 2 more minutes.a
→ Using the homogeneous mixture obtained above, tablets are compressed in a hardness range of 60 - 140 N.

## Claims

1. A water-dispersible tablet formulation consisting of a powder phase and a granular phase comprising deferasirox or a pharmaceutically acceptable salt thereof and colloidal silicon dioxide, **characterized in that**
i. 50.0% by weight of the total amount of colloidal silicon dioxide is provided in the granular phase, and only water is used as granulation solvent, and
ii. 50.0% by weight of the total amount of colloidal silicon dioxide is provided in the powder phase,
provided that the granular phase does not include a binder.

2. The water-dispersible tablet formulation according to claims 1, further comprising a surfactant, binder, disintegrant, filler, and a lubricant.

3. The water-dispersible tablet formulation according to claims 1 to 2, wherein the surfactant is sodium lauryl sulfate in the powder phase in an amount of 3.00% to 5.00% by weight of the total amount of the tablet.

4. The water-dispersible tablet formulation according to claims 1 to 3, wherein the binder is polyvinylpyrrolidone-vinyl acetate copolymer.

5. The water-dispersible tablet formulation according to claim 4, wherein the amount of polyvinylpyrrolidone-vinyl acetate copolymer in the powder phase is an amount of 1.00% to 3.00% by weight of the total amount of the tablet.

6. The water-dispersible tablet formulation according to any of the claims 1 to 5, wherein the tablet hardness is between 60 - 140 N.

7. The water-dispersible tablet formulation according to any of the claims 1 to 6, wherein the granular phase of the tablet comprises deferasirox, 50% by weight of total amount of colloidal silicon dioxide, and 55% by weight of total amount of crospovidone.

8. The water-dispersible tablet formulation according to any of the claims 1 to 7, wherein the powder phase of the tablet comprises 50% by weight of the total amount of colloidal silicon dioxide, sodium lauryl sulfate, polyvinylpyrrolidone-vinyl acetate copolymer, 45% by weight of the total amount of crospovidone, spray-dried lactose monohydrate, microcrystalline cellulose and magnesium stearate.

9. The pharmaceutical formulation according to any of the preceding claims, comprising the following ingredients:
a. granular phase of the tablet:
i. deferasirox in an amount of 26.00% to 29.00% of the total weight of the tablet,
ii. crospovidone in an amount of 18.00% to 21.00% of the total weight of the tablet,
iii. colloidal silicon dioxide in an amount of 0.01 to 0.50% of the total weight of the tablet,
b. powder phase of the tablet:
i. crospovidone in an amount of 17.00% to 20.00% of the total weight of the tablet,
ii. spray-dried lactose monohydrate in an amount of 8.00% to 11.00% of the total weight of the tablet,
iii. microcrystalline cellulose in an amount of 17.00% to 20.00% of the total weight of the tablet,
iv. polyvinylpyrrolidone-vinyl acetate copolymer in an amount of 1.00% to 3.00% of the total weight of the tablet,
v. sodium lauryl sulfate in an amount of 3.00% to 5.00% of the total weight of the tablet,
vi. colloidal silicon dioxide in an amount of 0.01% to 0.50% of the total weight of the tablet,
vii. magnesium stearate in an amount of 0.10% to 1.00% of the total weight of the tablet.
where the granular phase of the tablet does not include a binder.

10. A production process for the pharmaceutical formulation according to any of the preceding claims, comprising the following steps,
i. granulating deferasirox, 50.0% by weight of the total amount of colloidal silicon dioxide, and at least one excipient using water, where said excipient is not a binder,
ii. drying the wet granules obtained according to step (i) in a drying oven to give the granular phase of the tablet,
iii. mixing 50.0% by weight of the total amount of colloidal silicon dioxide and at least one excipient together to give the powder phase of the tablet,
iv. adding the powder phase of step (iii) to the granular phase of step (ii).

11. The production process according to claim 10, comprising the following steps,
i. granulating deferasirox, 50.0% by weight of the total amount of colloidal silicon dioxide, and at least one excipient using water,
ii. drying the wet granules obtained according to step (i) in a drying oven to give the granular phase of the tablet,
iii. mixing 50.0% by weight of the total amount of colloidal silicon dioxide, sodium lauryl sulfate, polyvinylpyrrolidone-vinyl acetate copolymer and at least one excipient together to give the powder phase of the tablet,
iv. adding the powder phase of step (iii) to the granular phase of step (ii).

## Patentansprüche

1. Wasserdispergierbare Tablettenformulierung, bestehend aus einer Pulverphase und einer Granulatphase, umfassend Deferasirox oder ein pharmazeutisch verträgliches Salz davon und kolloidales Siliziumdioxid, **dadurch gekennzeichnet, dass**
i. 50,0 Gew.-% der Gesamtmenge an kolloidalem Siliziumdioxid in Granulatphase bereitgestellt werden, und nur Wasser als Granulierungsmittel verwendet wird, und
ii. 50,0 Gew.-% der Gesamtmenge an kolloidalem Siliziumdioxid in Pulverphase bereitgestellt werden,
mit der Maßgabe, dass die Granulatphase kein Bindemittel enthält.

2. Wasserdispergierbare Tablettenformulierung nach Anspruch 1, des Weiteren umfassend ein Tensid, ein Bindemittel, ein Zerfallsmittel, einen Füllstoff und ein Gleitmittel.

3. Wasserdispergierbare Tablettenformulierung nach den Ansprüchen 1 bis 2, bei der das Tensid Natriumlaurylsulfat in der Pulverphase in einer Menge von 3,00 Gew.-% bis 5,00 Gew.-% der Gesamtmenge der Tablette ist.

4. Wasserdispergierbare Tablettenformulierung nach den Ansprüchen 1 bis 3, bei der das Bindemittel Polyvinylpyrrolidon-Vinylacetat-Copolymer ist.

5. Wasserdispergierbare Tablettenformulierung nach Anspruch 4, bei der die Menge an Polyvinylpyrrolidon-Vinylacetat-Copolymer in der Pulverphase eine Menge von 1,00 Gew.-% bis 3,00 Gew.-% der Gesamtmenge der Tablette ist.

6. Wasserdispergierbare Tablettenformulierung nach einem der Ansprüche 1 bis 5, bei der die Tablettenhärte zwischen 60 N und 140 N liegt.

7. Wasserdispergierbare Tablettenformulierung nach einem der Ansprüche 1 bis 6, bei der die Granulatphase der Tablette Deferasirox, 50 Gew.-% der Gesamtmenge an kolloidalem Siliziumdioxid und 55 Gew.-% der Gesamtmenge an Crospovidon umfasst.

8. Wasserdispergierbare Tablettenformulierung nach einem der Ansprüche 1 bis 7, bei der die Pulverphase der Tablette 50 Gew.-% der Gesamtmenge an kolloidalem Siliziumdioxid, Natriumlaurylsulfat, Polyvidonpyrrolidon-Vinylacetet-Copolymer, 45 Gew.-% der Gesamtmenge an Crospovidon, sprühgetrocknetem Lactosemonohydrat, mikrokristalliner Cellulose und Magnesiumstearat umfasst.

9. Pharmazeutische Formulierung nach einem der vorhergehenden Ansprüche, umfassend die folgenden Inhaltsstoffe:
a. Granulatphase der Tablette:
i. Deferasirox in einer Menge von 26,00 Gew.-% bis 29,00 Gew.-% des Gesamtgewichts der Tablette,
ii. Crospovidon in einer Menge von 18,00 Gew.-% bis 21,00 Gew.-% des Gesamtgewichts der Tablette,
iii. kolloidales Siliziumdioxid in einer Menge von 0,01 Gew.-% bis 0,50 Gew.-% des Gesamtgewichts der Tablette,
b. Pulverphase der Tablette:
i. Crospovidon in einer Menge von 17,00 Gew.-% bis 20,00 Gew.-% des Gesamtgewichts der Tablette,
ii. sprühgetrocknetes Lactosemonohydrat in einer Menge von 8,00 Gew.-% bis 11,00 Gew.-% des Gesamtgewichts der Tablette,
iii. mikrokristalline Cellulose in einer Menge von 17,00 Gew.-% bis 20,00 Gew.-% des Gesamtgewichts der Tablette,
iv. Polyvidonpyrrolidon-Vinylacetet-Copolymer in einer Menge von 1,00 Gew.-% bis 3,00 Gew.-% des Gesamtgewichts der Tablette,
v. Natriumlaurylsulfat in einer Menge von 3,00 Gew.-% bis 5,00 Gew.-% des Gesamtgewichts der Tablette,
vi. kolloidales Siliziumdioxid in einer Menge von 0,01 Gew.-% bis 0,50 Gew.-% des Gesamtgewichts der Tablette,
vii. Magnesiumstearat in einer Menge von 0,10 Gew.-% bis 1,00 Gew.-% des Gesamtgewichts der Tablette,
wobei die Granulatphase der Tablette kein Bindemittel enthält.

10. Herstellungsverfahren für die pharmazeutische Formulierung nach einem der vorhergehenden Ansprüche, umfassend die folgenden Schritte:
i. Granulieren von Deferasirox, 50,0 Gew.-% der Gesamtmenge an kolloidalem Siliziumdioxid, und mindestens einem Hilfsstoff unter Verwendung von Wasser, wobei der Hilfsstoff kein Bindemittel ist,
ii. Trocknen des gemäß Schritt (i) erhaltenen nassen Granulats in einem Trockenofen, um die Granulatphase der Tablette zu erhalten,
iii. Zusammenmischen von 50,0 Gew.-% der Gesamtmenge an kolloidalem Siliziumdioxid und mindestens einem Hilfsstoff, um die Pulverphase der Tablette zu erhalten,
iv. Hinzufügen der Pulverphase von Schritt (iii) zur Granulatphase von Schritt (ii).

11. Herstellungsverfahren nach Anspruch 10, umfassend die folgenden Schritte:
i. Granulieren von Deferasirox, 50,0 Gew.-% der Gesamtmenge an kolloidalem Siliziumdioxid und mindestens einem Hilfsstoff unter Verwendung von Wasser,
ii. Trocknen des gemäß Schritt (i) erhaltenen nassen Granulats in einem Trockenofen, um die Granulatphase der Tablette zu erhalten,
iii. Zusammenmischen von 50,0 Gew.-% der Gesamtmenge an kolloidalem Siliziumdioxid, Natriumlaurylsulfat, Polyvinylpyrrolidon-Vinylacetat-Copolymer und mindestens einem Hilfsstoff, um die Pulverphase der Tablette zu erhalten,
iv. Hinzufügen der Pulverphase von Schritt (iii) zur Granulatphase von Schritt (ii).

## Revendications

1. Formulation de comprimé dispersible dans l'eau, consistant en une phase pulvérulente et une phase granulaire comprenant du déférasirox ou un sel pharmaceutiquement acceptable de celui-ci et du dioxyde de silicium colloïdal, **caractérisée par le fait que** :
i. 50,0 % en poids de la quantité totale de dioxyde de silicium colloïdal sont fournis dans la phase granulaire, et seule de l'eau est utilisée comme solvant de granulation ; et
ii. 50,0 % en poids de la quantité totale de dioxyde de silicium colloïdal sont fournis dans la phase pulvérulente,
à la condition que la phase granulaire ne comprenne pas de liant.

2. Formulation de comprimé dispersible dans l'eau selon la revendication 1, comprenant en outre un tensio-actif, un liant, un désintégrant, une charge et un lubrifiant.

3. Formulation de comprimé dispersible dans l'eau selon l'une des revendications 1 et 2, dans laquelle le tensio-actif est le lauryl sulfate de sodium dans la phase pulvérulente dans une quantité de 3,00 % à 5,00 % en poids de la quantité totale du comprimé.

4. Formulation de comprimé dispersible dans l'eau selon l'une des revendications 1 à 3, dans laquelle le liant est un copolymère polyvinylpyrrolidone-acétate de vinyle.

5. Formulation de comprimé dispersible dans l'eau selon la revendication 4, dans laquelle la quantité de copolymère polyvinylpyrrolidone-acétate de vinyle dans la phase pulvérulente est une quantité de 1,00 % à 3,00 % en poids de la quantité totale du comprimé.

6. Formulation de comprimé dispersible dans l'eau selon l'une quelconque des revendications 1 à 5, dans laquelle la dureté du comprimé est entre 60-140 N.

7. Formulation de comprimé dispersible dans l'eau selon l'une quelconque des revendications 1 à 6, dans laquelle la phase granulaire du comprimé comprend du déférasirox, 50 % en poids de la quantité totale de dioxyde de silicium colloïdal, et 55 % en poids de la quantité totale de crospovidone.

8. Formulation de comprimé dispersible dans l'eau selon l'une quelconque des revendications 1 à 7, dans laquelle la phase pulvérulente du comprimé comprend 50 % en poids de la quantité totale de dioxyde de silicium colloïdal, du lauryl sulfate de sodium, un copolymère polyvinylpyrrolidone-acétate de vinyle, 45 % en poids de la quantité totale de crospovidone, du lactose monohydraté séché par pulvérisation, de la cellulose microcristalline et du stéarate de magnésium.

9. Formulation pharmaceutique selon l'une quelconque des revendications précédentes, comprenant les ingrédients suivants :
a. phase granulaire du comprimé :
i. déférasirox dans une quantité de 26,00 % à 29,00 % du poids total du comprimé ;
ii. crospovidone dans une quantité de 18,00 % à 21,00 % du poids total du comprimé ;
iii. dioxyde de silicium colloïdal dans une quantité de 0,01 à 0,50 % du poids total du comprimé ;
b. phase pulvérulente du comprimé :
i. crospovidone dans une quantité de 17,00 % à 20,00% du poids total du comprimé ;
ii. lactose monohydraté séché par pulvérisation dans une quantité de 8,00 % à 11,00 % du poids total du comprimé ;
iii. cellulose microcristalline dans une quantité de 17,00 % à 20,00 % du poids total du comprimé ;
iv. copolymère polyvinylpyrrolidone-acétate de vinyle dans une quantité de 1,00 % à 3,00 % du poids total du comprimé ;
v. lauryl sulfate de sodium dans une quantité de 3,00 % à 5,00 % du poids total du comprimé ;
vi. dioxyde de silicium colloïdal dans une quantité de 0,01 % à 0,50 % du poids total du comprimé ;
vii. stéarate de magnésium dans une quantité de 0,10 % à 1,00 % du poids total du comprimé,
la phase granulaire du comprimé ne comprenant pas de liant.

10. Procédé de production pour la formulation pharmaceutique selon l'une quelconque des revendications précédentes, comprenant les étapes suivantes :
i. granuler du déférasirox, 50,0 % en poids de la quantité totale de dioxyde de silicium colloïdal et au moins un excipient en utilisant de l'eau, ledit excipient n'étant pas un liant ;
ii. sécher les granulés humides obtenus conformément à l'étape (i) dans un four de séchage pour donner la phase granulaire du comprimé ;
iii. mélanger ensemble 50,0 % en poids de la quantité totale de dioxyde de silicium colloïdal et au moins un excipient pour donner la phase pulvérulente du comprimé ;
iv. ajouter la phase pulvérulente de l'étape (iii) à la phase granulaire de l'étape (ii).

11. Procédé de production selon la revendication 10, comprenant les étapes suivantes :
i. granuler du déférasirox, 50,0% en poids de la quantité totale de dioxyde de silicium colloïdal et au moins un excipient en utilisant de l'eau ;
ii. sécher les granulés humides obtenus conformément à l'étape (i) dans un four de séchage pour donner la phase granulaire du comprimé ;
iii. mélanger ensemble 50,0 % en poids de la quantité totale du dioxyde de silicium colloïdal, le lauryl sulfate de sodium, le copolymère polyvinylpyrrolidone-acétate de vinyle et au moins un excipient pour donner la phase pulvérulente du comprimé ;
iv. ajouter la phase pulvérulente de l'étape (iii) à la phase granulaire de l'étape (ii).
